# EUROPEAN PATENT APPLICATION

(11) **EP 1 762 218 A1**
(43) Date of publication of application: **14.03.2007**
(21) Application number: 05018739.2
(22) Date of filing: 30.08.2005
(51) Int. Cl.: A61K 8/41, A61Q 5/10, A61K 8/49, C07C 233/48

(54) **4-aminophenol derivatives and colorants comprising these compounds**

(71) Applicant: Wella Aktiengesellschaft, 64274 Darmstadt (DE)
(72) Inventor: Pasquier, Cécile, 1723 Marly (CH); Duc-Reichlin, Nadia, 1470 Lully (CH); Buclin, Veronique, 1638 Morlon (CH); Braun, Hans-Jürgen, 3182 Ueberstorf (CH)

(57) **Abstract**

4-Aminophenol derivative of the general formula (I) or physiologically compatible, water-soluble salt thereof, 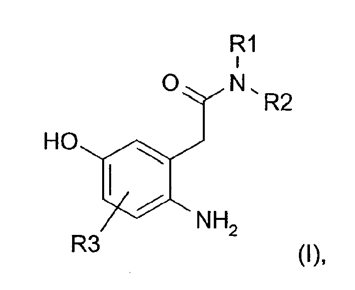 and agent comprising these compounds for the oxidative dyeing of keratin fibers.

## Description

The present invention relates to agents for the dyeing of keratin fibers based on a developer substance-coupler substance combination which comprise 4-aminophenol derivatives as developer substance, and to novel 4-aminophenol derivatives.

In the field of dyeing keratin fibers, in particular hair coloring, oxidation dyes have achieved significant importance. The coloration arises here as a result of the reaction of certain developer substances with certain coupler substances in the presence of a suitable oxidizing agent. The developer substances used here are, in particular, 2,5-diaminotoluene, 2,5-diaminophenylethyl alcohol, p-aminophenol and 1,4-diaminobenzene, while examples of coupler substances are resorcinol, 4-chlororesorcinol, 1-naphthol, 3-aminophenol and derivatives of m-phenylenediamine.

Besides dyeing to the desired intensity, numerous additional requirements are placed on oxidation dyes which are used for coloring human hair. For example, the dyes must be acceptable from a toxicological and dermatological point of view and the hair colorations achieved must have good light fastness, permanent wave fastness, acid fastness and rubbing fastness. However, in any case, such colorations must remain stable over a period of at least 4 to 6 weeks without being affected by light, rubbing and chemical agents. Furthermore, it is required that, by combining suitable developer substances and coupler substances, a broad palette of different color nuances can be produced.

Using the currently used colorants, as are described, for example, in the monograph by K.H. Schrader "Grundlagen und Rezepturen der Kosmetika" [Fundamentals and formulations of cosmetics], 2nd edition (1989), pages 784-799, it is, however, not possible to satisfy the above mentioned requirements in all aspects. Therefore there is still a need for novel developer substances which satisfy the above mentioned requirements to a particular degree.

In this regard, it has now surprisingly been found that certain 4-aminophenol derivatives according to the general formula (I) satisfy the requirements placed on developer substances to a particularly high degree. Thus, when these developer substances are used with most known coupler substances, color-rich shades are obtained which are extraordinarily light fast and wash fast.

The present invention therefore provides an agent for the oxidative dyeing of keratin fibers, such as, for example, hair, furs, feathers or wool, in particular human hair, based on a developer substance-coupler substance combination which comprises (as developer substance) at least one 4-aminophenol derivative of the formula (I) or its physiologically compatible, water-soluble salt, 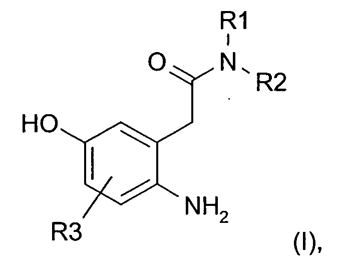 in which
**R1** and **R2**, independently of one another, are hydrogen, a saturated (C₁-C₆)-alkyl group, an unsaturated (C₂-C₆)-alkyl group, a (C₂-C₆)-hydroxyalkyl group, a (C₃-C₆)-dihydroxyalkyl group, a (C₁-C₄)-alkoxy-(C₁-C₄)-alkyl group, a (C₂-C₄)-hydroxyalkyl-(C₁-C₄)-alkoxy group, a (C₂-C₆)-aminoalkyl group, a (C₁-C₄)-alkylamino-(C₁-C₄)-alkyl group, a di(C₁-C₄)-alkylamino-(C₁-C₄)-alkyl group, a (C₂-C₆)-acetylaminoalkyl group, a (C₁-C₆)-cyanoalkyl group, a (C₁-C₆)-carboxyalkyl group, a (C₁-C₆)-amino-carbonylalkyl group, a substituted or unsubstituted phenyl group, a substituted or unsubstituted benzyl group, a pyridinylmethyl group, a furfuryl group, a hydrogenated furfuryl group, a substituted pyridinyl group, or R1 and R2, together with the nitrogen atom, form a heterocyclic ring of the formula 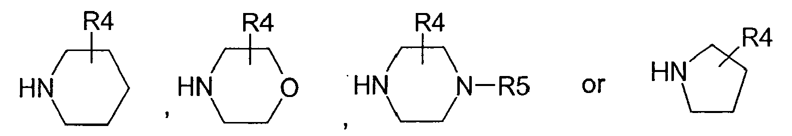 **R3** is hydrogen, a halogen atom, a C₁-C₆-alkyl group, a C₁-C₆-hydroxyalkyl group or a C₁-C₆-alkoxy group;
**R4** is one or more hydrogen atoms, hydroxyl groups, carboxyl groups, aminocarbonyl groups or hydroxymethyl groups; and
**R5** is hydrogen or a (C₁-C₆)-alkyl group.

Preference is given to compounds of the formula (I) in which R3 is hydrogen.

Compounds of the formula (I) which may be mentioned are, for example, the following compounds:
2-(2-amino-5-hydroxyphenyl)acetamide,
2-(2-amino-5-hydroxyphenyl)-N-methylacetamide,
2-(2-amino-5-hydroxyphenyl)-N-ethylacetamide,
2-(2-amino-5-hydroxyphenyl)-N-propylacetamide,
2-(2-amino-5-hydroxyphenyl)-N-isopropylacetamide,
2-(2-amino-5-hydroxyphenyl)-N-butylacetamide,
2-(2-amino-5-hydroxyphenyl)-N-(2-hydroxyethyl)acetamide,
2-(2-amino-5-hydroxyphenyl)-N-(3-hydroxypropyl)acetamide,
2-(2-amino-5-hydroxyphenyl)-N-(2,3-dihydroxypropyl)acetamide,
2-(2-amino-5-hydroxyphenyl)-N-(2-methoxyethyl)acetamide,
2-(2-amino-5-hydroxyphenyl)-N-(3-methoxypropyl)acetamide,
2-(2-amino-5-hydroxyphenyl)-N-(cyanomethyl)acetamide,
N-(2-aminoethyl)-2-(2-amino-5-hydroxyphenyl)acetamide,
N-(2-aminopropyl)-2-(2-amino-5-hydroxyphenyl)acetamide,
2-(2-amino-5-hydroxyphenyl)-N-cyclopentylacetamide,
2-(2-amino-5-hydroxyphenyl)-N,N-dimethylacetamide,
2-(2-amino-5-hydroxyphenyl)-N,N-diethylacetamide,
2-(2-amino-5-hydroxyphenyl)-N,N-dipropylacetamide,
2-(2-amino-5-hydroxyphenyl)-N,N-dibutylacetamide,
2-(2-amino-5-hydroxyphenyl)-N,N-bis(2-hydroxyethyl)acetamide,
2-(2-amino-5-hydroxyphenyl)-N,N-bis(3-hydroxypropyl)acetamide,
2-(2-amino-5-hydroxyphenyl)-N-(tetrahydro-2-furanylmethyl)acetamide,
2-(2-amino-5-hydroxyphenyl)-N-(2-furanylmethyl)acetamide,
2-(2-amino-5-hydroxyphenyl)-N-(2-pyridinylmethyl)acetamide,
2-(2-amino-5-hydroxyphenyl)-N-benzylacetamide,
4-amino-3-[2-oxo-2-(1-pyrrolidinyl)ethyl]phenol,
4-amino-3-[2-oxo-2-(1-piperidinyl)ethyl]phenol,
4-amino-3-[2-(4-morpholinyl)-2-oxoethyl]phenol,
4-amino-3-[2-(4-methyl-1-piperazinyl)-2-oxoethyl]phenol,
1-[(2-amino-5-hydroxyphenyl)acetyl]-3-pyrrolidinol,
1-[(2-amino-5-hydroxyphenyl)acetyl]-3-piperidinol,
2-(2-amino-5-hydroxyphenyl)-N-phenylacetamide,
2-(2-amino-5-hydroxyphenyl)-N-(4-hydroxyphenyl)acetamide,
2-(2-amino-5-hydroxyphenyl)-N-(3-hydroxyphenyl)acetamide,
2-(2-amino-5-hydroxyphenyl)-N-(2-hydroxyphenyl)acetamide,
2-(2-amino-5-hydroxyphenyl)-N-(4-methoxyphenyl)acetamide,
2-(2-amino-5-hydroxyphenyl)-N-(3-methoxyphenyl)acetamide,
2-(2-amino-5-hydroxyphenyl)-N-(2-methoxyphenyl)acetamide,
2-(2-amino-5-hydroxyphenyl)-N-[4-(hydroxymethyl)phenyl]acetamide,
2-(2-amino-5-hydroxyphenyl)-N-(2,4-dimethoxyphenyl)acetamide,
2-(2-amino-5-hydroxyphenyl)-N-[4-(dimethylamino)phenyl]acetamide,
2-(2-amino-5-hydroxyphenyl)-N-[3-(dimethylamino)phenyl]acetamide,
2-(2-amino-5-hydroxyphenyl)-N-[2-(dimethylamino)phenyl]acetamide,
2-(2-amino-5-hydroxyphenyl)-N-(4-chlorophenyl)acetamide,
2-(2-amino-5-hydroxyphenyl)-N-(3-chlorophenyl)acetamide,
2-(2-amino-5-hydroxyphenyl)-N-(2-chlorophenyl)acetamide,
2-(2-amino-5-hydroxyphenyl)-N-(4-bromophenyl)acetamide,
2-(2-amino-5-hydroxyphenyl)-N-(3-bromophenyl)acetamide,
2-(2-amino-5-hydroxyphenyl)-N-(2-bromophenyl)acetamide,
2-(2-amino-5-hydroxyphenyl)-N-(4-fluorophenyl)acetamide,
2-(2-amino-5-hydroxyphenyl)-N-(3-fluorophenyl)acetamide,
2-(2-amino-5-hydroxyphenyl)-N-(2-fluorophenyl)acetamide,
2-(2-amino-5-hydroxyphenyl)-N-(4-methylphenyl)acetamide,
2-(2-amino-5-hydroxyphenyl)-N-(3-methylphenyl)acetamide,
2-(2-amino-5-hydroxyphenyl)-N-(2-methylphenyl)acetamide,
2-(2-amino-5-hydroxyphenyl)-N-(4-trifluoromethylphenyl)acetamide,
2-(2-amino-5-hydroxyphenyl)-N-(3-trifluoromethylphenyl)acetamide,
2-(2-amino-5-hydroxyphenyl)-N-(2-trifluoromethylphenyl)acetamide,
2-(2-amino-5-hydroxyphenyl)-N-(4-nitrophenyl)acetamide,
2-(2-amino-5-hydroxyphenyl)-N-(3-nitrophenyl)acetamide,
2-(2-amino-5-hydroxyphenyl)-N-(2-nitrophenyl)acetamide,
2-(2-amino-5-hydroxyphenyl)-N-(4-cyanophenyl)acetamide,
2-(2-amino-5-hydroxyphenyl)-N-(3-cyanophenyl)acetamide,
2-(2-amino-5-hydroxyphenyl)-N-(2-cyanophenyl)acetamide,
2-(2-amino-5-hydroxyphenyl)-N-(4-pyridinyl)acetamide,
2-(2-amino-5-hydroxyphenyl)-N-(3-pyridinyl)acetamide and
2-(2-amino-5-hydroxyphenyl)-N-(2-pyridinyl)acetamide.

The compounds of the formula (I) can be used either in the form of free bases or in the form of their physiologically compatible salts with inorganic or organic acids, such as, for example, hydrochloric acid, sulfuric acid, phosphoric acid, acetic acid, propionic acid, lactic acid or citric acid.

The 4-aminophenol derivatives of the formula (I) are present in the colorant according to the invention in a total amount of from about 0.005 to 20 percent by weight, preference being given to an amount of from about 0.01 to 8 percent by weight and in particular 0.1 to 5 percent by weight.

Suitable coupler substances are preferably N-(3-dimethylaminophenyl)-urea, 2,6-diaminopyridine, 2-amino-4-[(2-hydroxyethyl)amino]anisole, 2,4-diamino-1-fluoro-5-methylbenzene, 2,4-diamino-1-methoxy-5-methylbenzene, 2,4-diamino-1-ethoxy-5-methylbenzene, 2,4-diamino-1-(2-hydroxyethoxy)-5-methylbenzene, 2,4-di[(2-hydroxyethyl)amino]-1,5-dimethoxybenzene, 2,3-diamino-6-methoxypyridine, 3-amino-6-methoxy-2-(methylamino)pyridine, 2,6-diamino-3,5-dimethoxypyridine, 3,5-diamino-2,6-dimethoxypyridine, 1,3-diaminobenzene, 2,4-diamino-1-(2-hydroxyethoxy)benzene, 1,3-diamino-4-(2,3-dihydroxypropoxy)benzene, 1,3-diamino-4-(3-hydroxypropoxy)benzene, 1,3-diamino-4-(2-methoxyethoxy)benzene, 2,4-diamino-1,5-di(2-hydroxyethoxy)benzene, 1-(2-aminoethoxy)-2,4-diaminobenzene, 2-amino-1-(2-hydroxyethoxy)-4-methylaminobenzene, 2,4-diaminophenoxyacetic acid, 3-[di(2-hydroxyethyl)amino]aniline, 4-amino-2-di[(2-hydroxyethyl)amino]-1-ethoxybenzene, 5-methyl-2-(1-methylethyl)phenol, 3-[(2-hydroxyethyl)amino]-aniline, 3-[(2-aminoethyl)amino]aniline, 1,3-di(2,4-diaminophenoxy)-propane, di(2,4-diaminophenoxy)methane, 1,3-diamino-2,4-dimethoxybenzene, 2,6-bis(2-hydroxyethyl)aminotoluene, 4-hydroxyindole, 3-dimethylaminophenol, 3-diethylaminophenol, 5-amino-2-methylphenol, 5-amino-4-fluoro-2-methylphenol, 5-amino-4-methoxy-2-methylphenol, 5-amino-4-ethoxy-2-methylphenol, 3-amino-2,4-dichlorophenol, 5-amino-2,4-dichlorophenol, 3-amino-2-methylphenol, 3-amino-2-chloro-6-methylphenol, 3-aminophenol, 2-[(3-hydroxyphenyl)amino]acetamide, 5-[(2-hydroxyethyl)amino]-4-methoxy-2-methylphenol, 5-[(2-hydroxyethyl)-amino]-2-methylphenol, 3-[(2-hydroxyethyl)amino]phenol, 3-[(2-methoxyethyl)amino]phenol, 5-amino-2-ethylphenol, 5-amino-2-methoxyphenol, 2-(4-amino-2-hydroxyphenoxy)ethanol, 5-[(3-hydroxypropyl)amino]-2-methylphenol, 3-[(2,3-dihydroxypropyl)amino]-2-methylphenol, 3-[(2-hydroxyethyl)amino]-2-methylphenol, 2-amino-3-hydroxypyridine, 2,6-dihydroxy-3,4-dimethylpyridine, 5-amino-4-chloro-2-methylphenol, 1-naphthol, 2-methyl-1-naphthol, 1,5-dihydroxynaphthalene, 1,7-dihydroxynaphthalene, 2,3-dihydroxynaphthalene, 2,7-dihydroxynaphthalene, 2-methyl-1-naphthol acetate, 1,3-dihydroxybenzene, 1-chloro-2,4-dihydroxybenzene, 2-chloro-1,3-dihydroxybenzene, 1,2-dichloro-3,5-dihydroxy-4-methylbenzene, 1,5-dichloro-2,4-dihydroxybenzene, 1,3-dihydroxy-2-methylbenzene, 3,4-methylenedioxyphenol, 3,4-methylenedioxyaniline, 5-[(2-hydroxyethyl)amino]-1,3-benzodioxol, 6-bromo-1-hydroxy-3,4-methylenedioxybenzene, 3,4-diaminobenzoic acid, 3,4-dihydro-6-hydroxy-1,4(2H)-benzoxazine, 6-amino-3,4-dihydro-1,4(2H)-benzoxazine, 3-methyl-1-phenyl-5-pyrazolone, 5,6-dihydroxyindole, 5,6-dihydroxyindoline, 5-hydroxyindole, 6-hydroxyindole, 7-hydroxyindole and 2,3-indolinedione.

Furthermore, in addition to the compounds of the formula (I), the colorant according to the invention can also comprise further known developer substances, for example 1,4-diaminobenzene (p-phenylenediamine), 1,4-diamino-2-methylbenzene (p-tolylenediamine), 1,4-diamino-2,6-dimethylbenzene, 1,4-diamino-3,5-diethylbenzene, 1,4-diamino-2,5-dimethylbenzene, 1,4-diamino-2,3-dimethylbenzene, 2-chloro-1,4-diaminobenzene, 1,4-diamino-2-(thiophen-2-yl)benzene, 1,4-diamino-2-(thiophen-3-yl)benzene, 4-(2,5-diaminophenyl)-2-((diethylamino)methyl)-thiophene, 2-chloro-3-(2,5-diaminophenyl)thiophene, 1,4-diamino-2-(pyridin-3-yl)benzene, 2,5-diaminobiphenyl, 2,5-diamino-4'-(1-methylethyl)-1,1'-biphenyl, 2,3',5-triamino-1,1'-biphenyl, 1,4-diamino-2-methoxymethylbenzene, 1,4-diamino-2-aminomethylbenzene, 1,4-diamino-2-((phenylamino)methyl)benzene, 1,4-diamino-2-((ethyl-(2-hydroxyethyl)-amino)methyl)benzene, 1,4-diamino-2-hydroxymethylbenzene, 1,4-diamino-2-(2-hydroxyethoxy)benzene, 2-(2-(acetylamino)ethoxy)-1,4-diaminobenzene, 4-phenylaminoaniline, 4-dimethylamino-aniline, 4-diethylaminoaniline, 4-dipropylaminoaniline, 4-[ethyl(2-hydroxyethyl)-amino]aniline, 4-[di(2-hydroxyethyl)amino]aniline, 4-[di(2-hydroxyethyl)-amino]-2-methylaniline, 4-[(2-methoxyethyl)amino]aniline, 4-[(3-hydroxypropyl)amino]aniline, 4-[(2,3-dihydroxypropyl)amino]aniline, 4-(((4-aminophenyl)methyl)amino)aniline, 4-[(4-aminophenylamino)-methyl]phenol, 1,4-diamino-N-(4-pyrrolidin-1-yl-benzyl)benzene, 1,3-di-hydroxy-2-((2-furylmethyl)aminomethyl)benzene, 1,4-diamino-N-thiophen-2-ylmethylbenzene, 1,4-diamino-N-furan-2-ylmethylbenzene, 1,4-diamino-N-thiophen-3-ylmethylbenzene, 1,4-diamino-N-benzylbenzene, 1,4-diamino-2-(1-hydroxyethyl)benzene, 1,4-diamino-2-(2-hydroxyethyl)-benzene, 1,4-diamino-2-(1-methylethyl)benzene, 1,3-bis[(4-aminophenyl)(2-hydroxyethyl)amino]-2-propanol, 1,4-bis[(4-aminophenyl)-amino]butane, 1,8-bis(2,5-diaminophenoxy)-3,6-dioxaoctane, 2,5-diamino-4'-hydroxy-1,1'-biphenyl, 2,5-diamino-2'-trifluoromethyl-1,1'-biphenyl, 2,4',5-triamino-1,1'-biphenyl, 4-aminophenol, 4-amino-3-methylphenol, 4-amino-3-(hydroxymethyl)phenol, 4-amino-3-fluorophenol, 4-methylaminophenol, 4-amino-2-(aminomethyl)phenol, 4-amino-2-(hydroxymethyl)phenol, 4-amino-2-fluorophenol, 4-amino-2-[(2-hydroxyethyl)amino]methylphenol, 4-amino-2-methylphenol, 4-amino-2-(methoxymethyl)phenol, 4-amino-2-(2-hydroxyethyl)phenol, 5-aminosalicylic acid, 2,5-diaminopyridine, 2,4,5,6-tetraaminopyrimidine, 2,5,6-triamino-4-(1H)-pyrimidone, 4,5-diamino-1-(2-hydroxyethyl)-1H-pyrazole, 4,5-diamino-1-(1-methylethyl)-1H-pyrazole, 4,5-diamino-1-[(4-methylphenyl)methyl]-1H-pyrazole, 1-[(4-chlorophenyl)methyl]-4,5-diamino-1H-pyrazole, 4,5-diamino-1-methyl-1H-pyrazole, 4,5-diamino-1-pentyl-1 H-pyrazole, 4,5-diamino-1-(phenylmethyl)-1H-pyrazole, 4,5-diamino-1-((4-methoxyphenyl)methyl-1H-pyrazole, 2-aminophenol, 2-amino-6-methylphenol, 2-amino-5-methylphenol, 1,2,4-trihydroxybenzene, 2,4-diaminophenol, 1,4-dihydroxybenzene and 2-(((4-aminophenyl)amino)methyl)-1,4-diaminobenzene.

The additional coupler substances and developer substances may be present in the colorant according to the invention in each case individually or in the mixture with one another, where the total amount of coupler substances and developer substances in the colorant according to the invention (based on the total amount of the colorant) is in each case about 0.005 to 20 percent by weight, preferably about 0.01 to 10 percent by weight, and in particular 0.1 to 5 percent by weight.

The total amount of the developer substance-coupler substance combination present in the colorant according to the invention is preferably about 0.01 to 20 percent by weight, particularly preferred is an amount of from about 0.02 to 15 percent by weight and especially 0.2 to 10 percent by weight. The developer substances and coupler substances are generally used in approximately equimolar amounts; however, it is not disadvantageous if the developer substances are present in this regard in a certain excess or deficit.

In addition, the colorant according to the invention can additionally comprise other color components, for example 6-amino-2-methylphenol and 2-amino-5-methylphenol, and also customary natural, nature-identical or synthetic direct dyes, for example triphenylmethane dyes, such as 4-[(4'-aminophenyl)(4'imino-2",5"-cyclohexadien-1"-ylidene)methyl]-2-methylaminobenzene monohydrochloride (C.I. 42 510) and 4-[(4'-amino-3'-methylphenyl)(4"-imino-3"-methyl-2",5"-cyclohexadien-1"-ylidene)-methyl]-2-methylaminobenzene monohydrochloride (C.I. 42 520), aromatic nitro dyes, such as 4-(2'-hydroxyethyl)aminonitrotoluene, 2-amino-4,6-dinitrophenol, 2-amino-5-(2'-hydroxyethyl)aminonitrobenzene, 2-chloro-6-(ethylamino)-4-nitrophenol, 4-chloro-N-(2-hydroxyethyl-2-nitroaniline, 5-chloro-2-hydroxy-4-nitroaniline, 2-amino-4-chloro-6-nitrophenol or 1-[(2'-ureidoethyl)amino-4-nitrobenzene, azo dyes, such as 6-[(4'-aminophenyl)azo]-5-hydroxynaphthalene-1-sulfonic acid sodium salt (C.I. 14 805) or dispersion dyes, such as, for example, 1,4-diaminoanthraquinone and 1,4,5,8-tetraaminoanthraquinone, and basic or acidic direct dyes. The colorant can comprise these color components in an amount of from about 0.1 to 4.0 percent by weight.

The coupler substances and developer substances and also the other color components, if they are bases, can of course also be used in the form of the physiologically compatible salts with organic or inorganic acids, such as, for example, hydrochloric acid, sulfuric acid or phosphoric acid, or - if they have aromatic OH groups - in the form of these salts with bases, for example as alkali metal phenoxides.

Moreover, if the colorants are to be used for dyeing hair, they may also comprise further customary cosmetic additives, for example antioxidants, such as ascorbic acid, thioglycolic acid and sodium sulfite, and perfume oils, complexing agents, wetting agents, emulsifiers, thickeners and care substances. The preparation form of the colorant according to the invention can, for example, be a solution, in particular an aqueous or aqueous-alcoholic solution. The particularly preferred preparation forms are, however, a cream, a gel or an emulsion. Their composition is a mixture of the dye components with the additives customary for such preparations.

Customary additives in solutions, creams, emulsions or gels are, for example, solvents, such as water, lower aliphatic alcohols, for example ethanol, propanol or isopropanol, glycerol or glycols, such as 1,2-propylene glycol, and also wetting agents or emulsifiers from the classes of anionic, cationic, amphoteric or nonionogenic surface-active substances, such as, for example, fatty alcohol sulfates, oxyethylated fatty alcohol sulfates, alkylsulfonates, alkylbenzenesulfonates, alkyltrimethylammonium salts, alkylbetaines, oxyethylated fatty alcohols, oxyethylated nonylphenols, fatty acid alkanolamides and oxyethylated fatty acid esters, also thickeners, such as higher fatty alcohols, starch, cellulose derivatives, petrolatum, paraffin oil and fatty acids, and also care substances, such as cationic resins, lanolin derivatives, cholesterol, pantothenic acid and betaine. The constituents mentioned are used in the amounts customary for such purposes, for example the wetting agents and emulsifiers in concentrations of from about 0.5 to 30 percent by weight, the thickeners in an amount of from about 0.1 to 25 percent by weight and the care substances in a concentration of from about 0.1 to 5 percent by weight.

Depending on the composition, the colorant of the invention can be weakly acidic, neutral or alkaline. In particular, it has a pH from 6.8 to 11.5.

According to the present invention for pH adjustement in the alkaline range the composition may further optionally comprise at least one source of alkalizing agent, preferably a source of ammonium ions and or ammonia. Any agent known in the art may be used such as alkanolamides for example monoethanolamine, diethanolamine, triethanolamine, monopropanolamine, dipropanolamine, tripropanolamine, 2-amino-2-methyl-1, 3-propanediol, 2-amino-2-methyl-1-propanol, and 2-amino-2-hydroxymethyl-1,3-propanediol and guanidium salts. Particularly, preferred alkalizing agents are those which provide a source of ammonium ions. Any source of ammonium ions is suitable for use herein. Preferred sources include ammonium chloride, ammonium sulphate, ammonium nitrate, ammonium phosphate, ammonium acetate, ammonium carbonate, ammonium hydrogen carbonate, ammonium carbamate, ammonium hydroxide, percarbonate salts, ammonia and mixtures thereof. Particularly preferred are ammonium carbonate, ammonium carbamate, ammonium hydrogen carbonate, ammonia and mixtures thereof or a mixture of ammonia and organic amines (particularly monoethanolamine or triethanolamine). The compositions of the present invention may comprise from about 0.1 % to about 10% by weight, preferably from about 0.5% to about 5%, most preferably from about 1 % to about 3% of an alkalizing agent, preferably ammonium ions.

For pH adjustment in the acidic range, an inorganic or organic acid, for example phosphoric acid, acetic acid, citric acid or tartaric acid, may be used.

The compositions according to the present invention may comprise at least one source of an oxidizing agent for developing the hair color.

Preferred oxidizing agents for use herein are water-soluble peroxygen oxidizing agents. "Water-soluble" as defined herein means that in standard condition at least 0.1g, preferably 1g, more preferably 10g of said oxidizing agent can be dissolved in 1 liter of deionized water. The oxidizing agents are valuable for the initial solubilisation and decolourisation of the melanin (bleaching) and accelerate the oxidation of the oxidative dye precursors (oxidative dyeing) in the hair shaft.

Any oxidizing agent known in the art may be utilized in the present invention. Preferred water-soluble oxidizing agents are inorganic peroxygen materials capable of yielding hydrogen peroxide in an aqueous solution. Water-soluble peroxygen oxidizing agents are well known in the art and include hydrogen peroxide, inorganic alkali metal peroxides such as sodium periodate and sodium peroxide and organic peroxides such as urea peroxide, melamine peroxide, and inorganic perhydrate salt bleaching compounds, such as the alkali metal salts of perborates, percarbonates, perphosphates, persilicates, persulphates and the like. These inorganic perhydrate salts may be incorporated as monohydrates, tetrahydrates etc. Alkyl and aryl peroxides, and or peroxidases may also be used. Mixtures of two or more such oxidizing agents can also be used if desired. The oxidizing agents may be provided in aqueous solution or as a powder which is dissolved prior to use. Preferred for use in the compositions according to the present invention are hydrogen peroxide, percarbonate, persulphates and combinations thereof.

According to the present invention the compositions comprise from about 0.1 % to about 15% by weight, preferably from about 1% to about 10% by weight, and most preferably from about 2% to about 7% by weight of an oxidizing agent.

Another preferred oxidizing agent for use herein is a source of peroxymonocarbonate ions. Preferably such a source is formed in situ from a source of hydrogen peroxide and a hydrogen carbonate ion source. Such an oxidizing agent has been found to be particularly effective at a pH of up to and including 9.5, preferably 7.5 to 9.5 more preferably about pH 9. Moreover, this system is also particularly effective in combination with a source of ammonia or ammonium ions. It has been found that this oxidizing agent can deliver improvements to the desired hair colour results particularly with regard to the delivery of high lift, whilst considerably reducing the odour, skin and scalp irritation and damage to the hair fibres.

Accordingly, any source of these ions may be utilized. Suitable sources for use herein include sodium, potassium, guanidine, arginine, lithium, calcium, magnesium, barium, ammonium salts of carbonate, carbamate and hydrocarbonate ions and mixtures thereof such as sodium carbonate, sodium hydrogen carbonate, potassium carbonate, potassium hydrogen carbonate, guanidine carbonate, guanidine hydrogen carbonate, lithium carbonate, calcium carbonate, magnesium carbonate, barium carbonate, ammonium carbonate, ammonium hydrogen carbonate and mixtures thereof. Percarbonate salts may also be utilized to provide both the source of carbonate ions and oxidizing agent. Preferred sources of carbonate ions, carbamate and hydrocarbonate ions are sodium hydrogen carbonate, potassium hydrogen carbonate, ammonium carbamate, and mixtures thereof.

According to the present invention the compositions comprise from about 0.1 % to about 15% by weight, preferably from about 1% to about 10% by weight, and most preferably from about 1 % to about 8% by weight of a hydrogencarbonate ion and from about 0.1 % to about 10% by weight, preferably from about 1% to about 7% by weight, and most preferably from about 2% to about 5% by weight of a source of hydrogen peroxide.

Especially preferred oxidants for developing the hair color are mainly hydrogen peroxide or a compound of addition of hydrogen peroxide to urea, melamine, sodium borate or sodium carbonate, in the form of a 3 to 12%, preferably 6%, aqueous solution, as well as air oxygen. When a 6% hydrogen peroxide solution is used as the oxidant, the weight ratio of hair colorant to oxidant is 5:1 to 2:1, and preferably 1:1. Larger amounts of oxidant are used primarily when the hair colorant contains a higher dye concentration or when stronger hair bleaching is desired at the same time. According to the present invention the compositions may further comprise a source of radical scavenger. As used herein the term radical scavenger refers to a species that can react with a reactive radical, preferably carbonate radicals, to convert the reactive radical by a series of fast reactions to a less reactive species. Suitable radical scavengers for use herein include compounds according to the general formula (I):

R¹-Y-C(H)(R³)-R⁴-(C(H)(R⁵)-Y-R⁶)ₙ (I)

wherein Y is NR², O, or S, preferably NR², n is 0 to 2, and wherein R⁴ is monovalent or divalent and is selected from: (a) substituted or unsubstituted, straight or branched, alkyl, mono- or poly-unsaturated alkyl, heteroalkyl, aliphatic, heteroaliphatic, or heteroolefinic systems, (b) substituted or unsubstituted, mono- or poly-cyclic aliphatic, aryl, or heterocyclic systems, or (c) substituted or unsubstituted, mono-, poly-, or per-fluoro alkyl systems; the systems of (a), (b) and (c) comprising from 1 to 12 carbon atoms and 0 to 5 heteroatoms selected from O, S, N, P, and Si; and wherein R⁴ can be connected to R³ or R⁵ to create a 5, 6 or 7 membered ring; and wherein R¹, R², R³, R⁵, and R⁶ are monovalent and are selected independently from: (a), (b) and (c) described herein above, or H.
Preferably, R⁴ is selected from: (a) substituted or unsubstituted, straight or branched, alkyl, heteroalkyl, aliphatic, heteroaliphatic, or heteroolefinic systems, (b) substituted or unsubstituted, mono- or poly-cyclic aliphatic, aryl, or heterocyclic systems, or (c) substituted or unsubstituted, mono-, poly-, or per-fluoro alkyl systems; more preferably R⁴ is selected from (a) substituted or unsubstituted, straight or branched, alkyl, heteroalkyl, aliphatic, or heteroaliphatic systems, (b) substituted or unsubstituted, aryl, or heterocyclic systems, or (c) substituted or unsubstituted, mono-, poly-, or per-fluoro alkyl systems; more preferably substituted or unsubstituted, straight or branched, alkyl, or heteroalkyl systems.
Preferably, the R⁴ systems of (a), (b), and (c), described herein above, comprise from 1 to 8 carbon atoms, preferably from 1 to 6, more preferably from 1 to 4 carbon atoms and from 0 to 3 heteroatoms; preferably from 0 to 2 heteroatoms; most preferably from 0 to 1 heteroatoms. Where the systems contain heteroatoms, preferably they contain 1 heteroatom. Preferred heteroatoms include O, S, and N; more preferred are O, and N; O being paricularly preferred.
Preferably, R¹, R², R³, R⁵, and R⁶ are selected independently from any of the systems defined for R⁴ above, and H.
In alternative embodiments, any of R¹, R², R³, R⁴, R⁵, and R⁶ groups are substituted. Preferably, the substituent(s) is selected from: (a) the group of C-linked monovalent substituents consisting of: (i) substituted or unsubstituted, straight or branched, alkyl, mono- or poly-unsaturated alkyl, heteroalkyl, aliphatic, heteroaliphatic, or heteroolefinic systems, (ii) substituted or unsubstituted, mono- or poly-cyclic aliphatic, aryl, or heterocyclic systems, or (iii) substituted or unsubstituted, monofluoroalkyl, polyfluoroalkyl or perfluoroalkyl systems; said systems of (i), (ii) and (iii) comprising from 1 to 10 carbon atoms and 0 to 5 heteroatoms selected from O, S, N, P, and Si; (b) the group of S-linked monovalent substituents consisting of SA¹, SCN, SO₂A¹, SO₃A¹, SSA¹, SOA¹, SO₂NA¹A², SNA¹A², and SONA¹A²; (c) the group of O-linked monovalent substituents consisting of OA¹, OCN and ONA¹A²; (d) the group of N-linked monovalent substituents consisting of NA¹A², (NA¹A²A³)⁺, NC, NA¹OA², NA¹SA², NCO, NCS, NO₂, N=NA¹, N=NOA¹, NA¹CN, NA¹NA²A³; (e) the group of monovalent substituents consisting of COOA¹, CON₃, CONA¹₂, CONA¹COA², C(=NA¹)NA¹A², CHO, CHS, CN, NC, and X; and (f) the group consisting fluoroalkyl monovalent substituents consisting of monofluoroalkyl, polyfluoroalkyl perfluoroalkyl systems comprising from 1 to 12 carbon atoms and 0 to 4 heteroatoms.
For the groups (b) to (e), described above, A¹, A², and A³ are monovalent and are independently selected from: (1) H, (2) substituted or unsubstituted, straight or branched, alkyl, monounsaturated or poly-unsaturated alkyl, heteroalkyl, aliphatic, heteroaliphatic or heteroolefinic systems,
(3) substituted or unsubstituted, monocyclic or polycyclic aliphatic, aryl or heterocyclic systems, or (4) substituted or unsubstituted, monofluoroalkyl, polyfluoroalkyl or perfluoroalkyl systems; said systems of (2), (3) and (4) comprising from 1 to 10 carbon atoms and 0 to 5 heteroatoms selected from O, S, N, P, and Si; and wherein X is a halogen selected from the group consisting of F, Cl, Br, and I.
Preferred substituents for use herein include those having a Hammett Sigma Para (σₚ) Value from -0.65 to +0.75, preferably from -0.4 to +0.5. Hammett Sigma Values are described in Advanced Organic Chemistry - Reactions, Mechanisms and Structure (Jerry March, 5th ed. (2001) at pages 368-375).
Alternative suitable radical scavengers for use herein are compounds according to the general formula (II) : 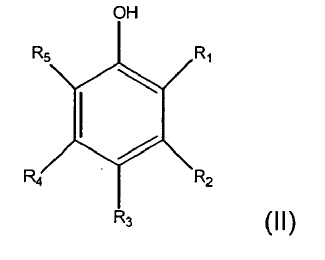 wherein R₁, R₂, R₃, R₄, and R₅ are each independently selected from H, COO⁻M⁺, Cl, Br, SO₃⁻M⁺, NO₂, OCH₃, OH or a C¹ to C¹⁰ primary or secondary alkyl and M is either H or alkali metal. Preferably, the above-described radical scavengers have a pKa of more than 8.5 to ensure protonation of the hydroxy goup.
Other suitable radical scavengers for use herein include those selected from group (III) benzylamine, imidazole, di-tert-butylhydroxytoluene, hydroquinone, guanine, pyrazine, piperidine, morpholine, methylmorpholine, 2-methyoxyethylamine, and mixtures thereof. Preferred radical scavengers according to the present invention are selected from the classes of alkanolamines, amino sugars, amino acids, esters of amino acids and mixtures thereof. Particularly preferred compounds are: monoethanolamine, 3-amino-1-propanol, 4-amino-1-butanol, 5-amino-1-pentanol, 1-amino-2-propanol, 1-amino-2-butanol, 1-amino-2-pentanol, 1-amino-3-pentanol, 1-amino-4-pentanol, 3-amino-2-methylpropan-1-ol, 1-amino-2-methylpropan-2-ol, 3-aminopropane-1,2-diol, glucosamine, N-acetylglucosamine, glycine, arginine, lysine, proline, glutamine, histidine, sarcosine, serine, glutamic acid, tryptophan, and mixtures thereof, and the salts such as the potassium, sodium and ammonium salts thereof and mixtures thereof. Especially preferred compounds are glycine, sarcosine, lysine, serine, 2 methoxyethylamine, glucosamine, glutamic acid, morpholine, piperidine, ethylamine, 3-amino-1-propanol and mixtures thereof.

The radical scavengers according to the present invention preferably have a molecular weight of less than about 500, preferably less than about 300, more preferably less than about 250 in order to facilitate penetration of the radical scavenger into the hair fibre. The compositions of the present invention preferably comprise from about 0.1% to about 10% by weight, preferably from about 1% to about 7% by weight of radical scavenger.
The radical scavenger is also preferably selected such that it is not an identical species as the alkalizing agent. According to one embodiment of the present invention the radical scavenger may be formed insitu in the hair dyeing compositions prior to application to the hair fibres.

To use the afore-described colorants for oxidative dyeing of hair, said colorants are mixed with an oxidant immediately before use, and the mixture is applied to hair in an amount sufficient for hair treatment which, depending on hair fullness, is generally from about 60 to 200 grams.

Suitable oxidants for developing the hair color are mainly hydrogen peroxide or a compound of addition of hydrogen peroxide to urea, melamine, sodium borate or sodium carbonate, in the form of a 3 to 12% by weight, preferably 6% by weight, aqueous solution, as well as air oxygen. When a 6% hydrogen peroxide solution is used as the oxidant, the weight ratio of hair colorant to oxidant is 5:1 to 2:1, and preferably 1:1. Larger amounts of oxidant are used primarily when the hair colorant contains a higher dye concentration or when stronger hair bleaching is desired at the same time.

The mixture is allowed to act on the hair at 15 to 50 °C for about 10 to 45 minutes and preferably for 30 minutes. The hair is then rinsed with water and dried. Optionally, this rinse can be followed with a shampoo wash, optionally followed by rinsing with a weak organic acid, for example citric or tartaric acid. The hair is then dried.

The hair colorants according to the invention with a content of 4-aminophenol derivatives of the formula (I) permit hair colorations with excellent color fastness, in particular with regard to light fastness, washing fastness and rubbing fastness. With regard to the coloring properties, the hair colorants according to the invention offer, depending on the nature and composition of the color components, a broad palette of different shades which ranges from blonde via brown, purple, violet to blue and black shades. The shades are characterized here by their particular color intensity. The very good coloring properties of the hair colorants according to the present application are further evident from the fact that these agents permit a coloring of gray, chemically non-predamaged hair without problems and with good coverage.

Another object of the present invention are the 4-aminophenol derivatives of the formula (I) described above or the physiologically tolerated water-soluble salts thereof.

The 4-aminophenol derivatives of the general formula (I) according to the invention can be prepared using known synthesis methods. The synthesis of the compounds according to the invention can, for example, be carried out as follows:
either a) by a one-carbon elongation of 3-hydroxy-6-nitrobenzaldehyde or its protected form via its dibromoalkene derivative (II), in analogy to the method described in Tetrahedron 58 (2002), pages 9925-9932, and final reduction/deprotection of the acetamide derivative of formula (III), according to Diagram 1, 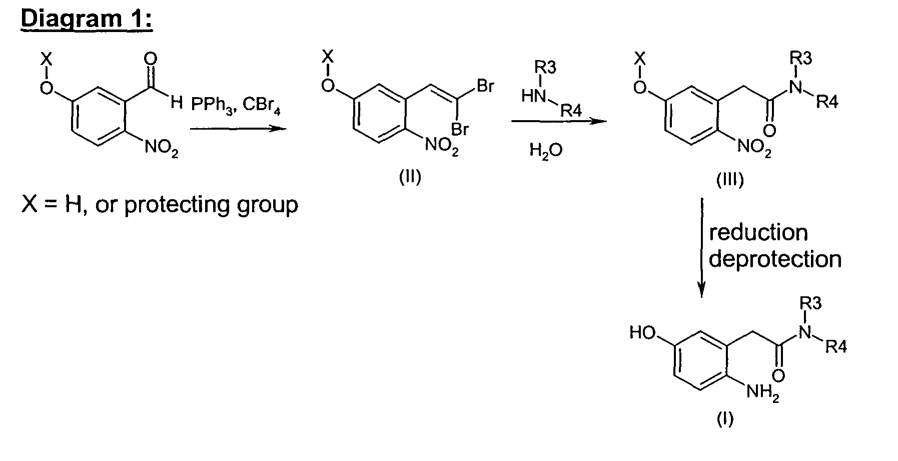
or b) by amide formation from the phenylacetic acid derivative (IV) using known coupling conditions and final reduction, deprotection (simultaneously when X stands for a benzyl group) of the acetamide derivative of formula (V), according to Diagram 2.

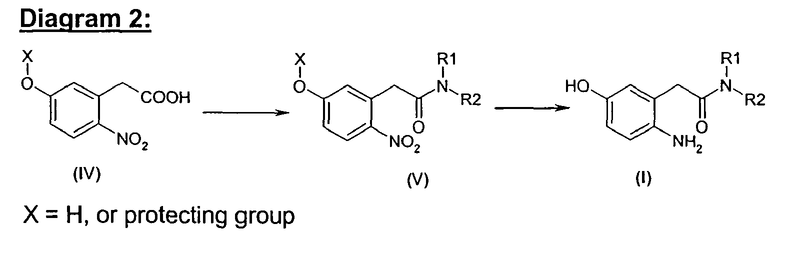

The 4-aminophenol derivatives of the formula (I) according to the invention are readily soluble in water and permit colorations with high color intensity and excellent color fastness, especially with regard to light fastness, washing fastness and rubbing fastness. The 4-aminophenol derivatives of the formula (I) also have excellent storage stability, especially as constituent of the colorants described above.

The following examples illustrate the object of the invention in more detail without limiting ist scope.

### Examples

### Examples 1 to 5: Synthesis of 4-aminophenol derivatives of the formula (I) (general synthesis procedure)

### A) Amide formation:

In a screw cap tube under argon 1 ml of dimethylformamide was added to a mixture of 0.125 g (0.43 mmol) of [5-(benzyloxy)-2-nitrophenyl]acetic acid, 0.234 g (0.56 mmol) of O-(1H-6-chlorobenzotriazol-1-yl)-1,1,3,3-tetramethyluronium hexafluorophosphate (HCTU) and 0.096 g (0.56 mmol) of 6-chloro-1-hydroxybenzotriazole (Cl-HOBT). The mixture was cooled in an ice bath, and 1.5 eq (0.64 mmol) of the corresponding amine and 0.11 g (0.87 mmol) of diisopropylethylamine were added. The reaction mixture was stirred at room temperature overnight, diluted with chloroform, and after addition of an aqueous solution of sodium carbonate extracted with chloroform. The organic layer was filtered through a pad of magnesium sulfate/silica gel and concentrated under reduced pressure. Purification was performed by column chromatography on silica gel to afford the [5-(benzyloxy)-2-nitrophenyl]acetamide derivative of formula (V) in 65 to 81 % yield.

### B) Reduction:

120 mg of [5-(benzyloxy)-2-nitrophenyl]acetamide derivative of formula (V) were hydrogenated with hydrogen in 10 ml of ethanol / tetrahydrofurane 1:1 in the presence of Pd/C 10 % (25 mg). After a reaction time of 2 to 6 hours the mixture was filtered through a pad of magnesium sulfate/celite and the filtrate was concentrated under reduced pressure. The crude product was purified by chromatography on silica gel to afford the product as solids or foams in 66 to 83 % yield.

### 1. 2-(2-amino-5-hydroxyphenyl)-N-(2-methoxyethyl)acetamide Amine used: methoxyethylamine

APCI-MS: 225 [M+H]⁺
¹H-NMR (300 MHz, DMSO): 8.34 (s, 1 H, OH); 8.09 (t, J=5.6, 1 H, NH-CH2); 6.49 (d, J=2.6, 1 H, H(6)); 6.48 (d, J=8,1, 1H, H(3)); 6.39 (dd, J=2.6, J=8.1, 1 H, H(4)); 4.49 (s, 2H, NH2); 3.33 (q, J=5.6, 2H, NH-CH2); 3.30 (s, 3H, O-CH3); 3.24 (s, 2H, CH2-C=O); 3.18 (t, J=5.6, 2H, CH2-O-CH3).

### 2. 4-amino-3-[2-(4-morpholinyl)-2-oxoethyl]phenol Amine used: morpholine

APCI-MS: 237 [M+H]⁺
¹H-NMR (300 MHz, DMSO): 8.37 (s, 1 H, OH); 6.50 (d, J=8.2, 1 H, H(3)); 6.44 (d, J=2.6, 1 H, H(6)); 6.40 (dd, J=2.6, J=8.2, 1 H, H(4)); 4.40 (s, 2H, NH2); 3.54-3.45 (m, 8H); 3.30 (s, 2H, CH2-C=O).

### 3. 1-[(2-amino-5-hydroxvphenyl)acetyl]-3-piperidinol Amine used: 3-piperidinol

APCI-MS: 251 [M+H]⁺
¹ H-NMR (300 MHz, DMSO): 8.36 (s, 1 H, OH); 6.49 (d, J=8.2, 1 H, H(3)); 6.44 (d, J=2.6, 1 H, H(6)); 6.40 (dd, J=2.6, J=8.2, 1 H, H(4)); 4.68 (d, J=4,0, 1 H, OH); 4.42 (s, 2H, NH2); 3.98-3.88 (m, 1 H); 3.70-3.60 (m, 2H); 3.43 (s, 2H, CH2-C=O); 3.18-2.98 (m, 2H); 1.72-1.52 (m, 2H); 1.30-1.10 (m, 2H).

### 4. 2-(2-amino-5-hydroxyphenyl)-N-(2-pyridinylmethyl)acetamide Amine used: 2-aminomethylpyridine

APCI-MS: 258 [M+H]⁺
¹H-NMR (300 MHz, DMSO): 8.58 (t, J=5.6, 1 H, NH); 8.50 (m, 1 H, H(6)-pyridinyl); 8.37 (s, 1 H, OH); 7.72 (dt, J=1.7, J=7.7, 1 H, H(5)-pyridinyl); 7.27-7.23 (m, 2H, H(3) and H(4)-pyridinyl); 6.54 (d, J=2.6, 1 H, H(6)); 6.51 (d, J=8.4, 1 H, H(3)); 6.41 (dd, J=2.6, J=8.4, 1 H, H(4)); 4.51 (s, 2H, NH2); 4.36 (d, J=5.6, CH2-NH); 3.33 (s, 2H, CH2-C=O).

### 5. 2-(2-amino-5-hydroxyphenyl)-N-(3-hydroxyphenyl)acetamide Amine used: 3-aminophenol

APCI-MS: 259 [M+H]⁺
¹H-NMR (300 MHz, DMSO): 9.97 (s, 1 H, OH); 9.33 (s, 1 H, OH); 8.39 (s, 1 H, NH); 7.16 (m, 2H); 7.05 (t, J=8.0, 1 H); 6.93 (d, J=8.0, 1 H); 6.56 (d, J=2.6, 1 H, H(6)); 6.53 (d, J=8.4, 1 H, H(3)); 6.43 (dd, J=2.6, J=8.4, 1 H, H(4)); 4.50 (s, 2H, NH2); 3.40 (s, 2H, CH2-C=O).

### Examples 6 to 15: Hair dyes (1:1 combinations)

| | |
|---|---|
| 1.25 mmol | 4-aminophenol of the formula (I) as described in examples 1 to 5 |
| 1.25 mmol | coupler substance as in table 1 |
| 10.0 g | lauryl ether sulfate (28 percent aqueous solution) |
| 9.0 g | ammonia (22 percent aqueous solution) |
| 7.8 g | ethanol |
| 0.3 g | ascorbic acid |
| 0.3 g | ethylenediaminotetraacetic acid disodium salt hydrate |
| ad 100.0 g | water, demineralized |

Directly prior to application 10 g of the above coloring solution were mixed with 10 g of a 6 percent hydrogen peroxide solution. The mixture was then applied to bleached hair. After a contact time of 30 minutes at 40°C, the hair was rinsed with water, washed with a standard commercial shampoo and dried. The resulting colorations are summarized in table 1.

**Table 1: Hair dyes**

| **Example No.** | **Developer substance of the formula (I)** | **Coupler substance** | | |
|---|---|---|---|---|
| | | **I. 3-Aminophenol** | **II. 5-Amino-2- methylphenol** | **III. 1,3-Dihydroxybenzene** |
| **6** | as in example **1** | light orange | orange | light blond |
| **7** | as in example **2** | light orange | orange | light blond |
| **8** | as in example **3** | light orange | orange | light blond |
| **9** | as in example **4** | light orange | orange | light blond |
| **10** | as in example **5** | light orange | orange | light blond |

| **IV. 2-amino-4-[(2- hydroxyethyl)-amino]anisole sulfate** | **V. 1,3-Bis(2,4- diaminophenoxy)- propane tetrahydrochloride** | **VI. 1,3-diamino-4-(2'-hydroxy-ethoxy)benzene dihydrochloride** | | |
|---|---|---|---|---|
| **11** | as in example **1** | violet | brown-violet | violet |
| **12** | as in example **2** | violet | brown-violet | violet |
| **13** | as in example **3** | violet | brown-violet | violet |
| **14** | as in example **4** | violet | brown-violet | violet |
| **15** | as in example **5** | violet | brown-violet | violet |

### Examples 16 to 50: Hair colorants (multi-combinations)

- Xg: 4-aminophenol of the formula (I) as described in examples 1 to 5 (**E1** to **E5** as in table 2)
- U g: developer substance **E8** to **E15** as in table 2
- Y g: coupler substance **K12** to **K36** as in table 4
- Z g: direct dyes **D1** to **D3** as in table 3
- 10.0 g: lauryl ether sulfate (28 percent aqueous solution)
- 9.0 g: ammonia (22 percent aqueous solution)
- 7.8 g: ethanol
- 0.3 g: ascorbic acid
- 0.3 g: ethylenediaminotetraacetic acid disodium salt hydrate
- ad 100.0 g: water, demineralized

Directly prior to application 30 g of the above coloring solution were mixed with 30 g of a 6 percent aqueous hydrogen peroxide solution. The mixture was then applied to bleached hair. After a contact time of 30 minutes at 40°C, the hair was rinsed with water, washed with a standard commercial shampoo and dried. The coloring results are summarized in table 5.

**Table 2:**

| **Developer substances** | |
|---|---|
| **E1** | 2-(2-amino-5-hydroxyphenyl)-N-[2-(methyloxy)ethyl]acetamide |
| **E2** | 4-amino-3-[2-(4-morpholinyl)-2-oxoethyl]phenol |
| **E3** | 1-[(2-amino-5-hydroxyphenyl)acetyl]-3-piperidinol |
| **E4** | 2-(2-amino-5-hydroxyphenyl)-N-(2-pyridinylmethyl)acetamide |
| **E5** | 2-(2-amino-5-hydroxyphenyl)-N-(3-hydroxyphenyl)acetamide |
| | |
| **E8** | 1,4-diaminobenzene |
| **E9** | 2,5-diaminophenylethanol sulfate |
| **E10** | 3-methyl-4-aminophenol |
| **E11** | 4-amino-2-aminomethylphenol dihydrochloride |
| **E12** | 4-aminophenol |
| **E13** | N,N-bis(2'-hydroxyethyl)-p-phenylendiamine sulfate |
| **E14** | 4,5-diamino-1-(2'-hydroxyethyl)pyrazole sulfate |
| **E15** | 2,5-diaminotoluene sulfate |

**Table 3:**

| **Direct dyes** | |
|---|---|
| **D1** | 2,6-diamino-3-((pyridin-3-yl)azo)pyridine |
| **D2** | 6-chloro-2-ethylamino-4-nitrophenol |
| **D3** | 2-amino-6-chloro-4-nitrophenol |

**Table 4:**

| **Coupler substances** | |
|---|---|
| **K12** | 2-amino-4-(2'-hydroxyethyl)aminoanisole sulfate |
| **K13** | 1,3-diamino-4-(2'-hydroxyethoxy)benzene sulfate |
| **K14** | 2,4-diamino-5-fluorotoluene sulfate |
| **K15** | 3-amino-6-methoxy-2-(methylamino)pyridine |
| **K16** | 3,5-diamino-2,6-dimethoxypyridine dihydrochloride |
| **K17** | 2,4-diamino-1-ethoxy-5-methylbenzene |
| **K18** | N-(3-dimethylamino)phenylurea |
| **K19** | 1,3-bis(2,4-diaminophenoxy)propane tetrahydrochloride |
| **K21** | 3-aminophenol |
| **K22** | 5-amino-2-methylphenol |
| **K23** | 3-amino-2-chloro-6-methylphenol |
| **K24** | 5-amino-4-fluoro-2-methylphenol sulfate |
| **K25** | 1-naphthol |
| **K31** | 1,3-dihydroxybenzene |
| **K32** | 2-methyl-1,3-dihydroxybenzene |
| **K33** | 1-chloro-2,4-dihydroxybenzene |
| **K34** | 4-(2'-hydroxyethyl)amino-1,2-methylenedioxybenzene hydrochloride |
| **K35** | 1,3-benzodioxol-5-ol |
| **K36** | 2-amino-5-methylphenol |

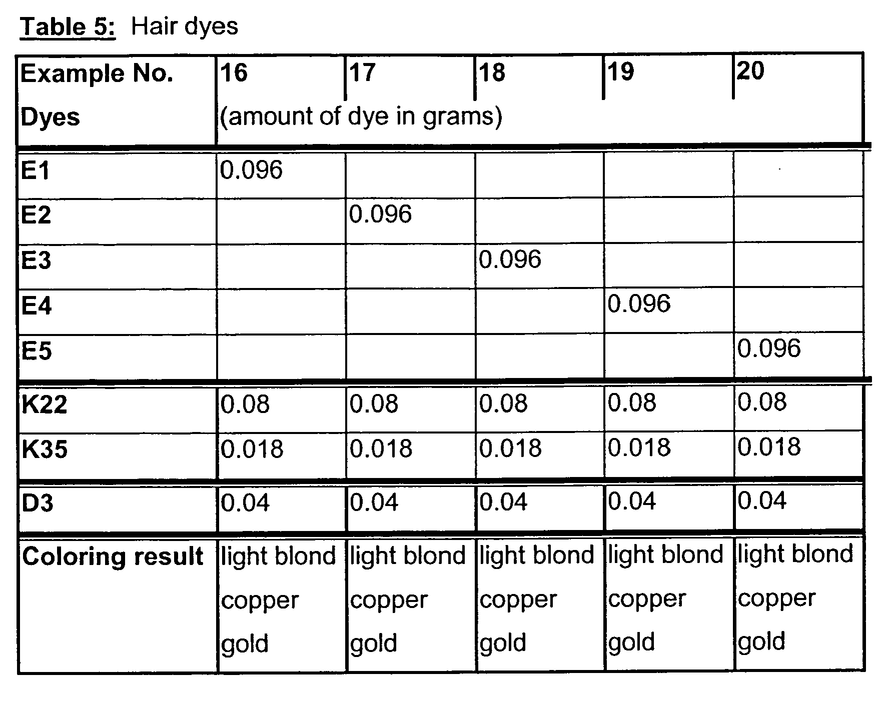

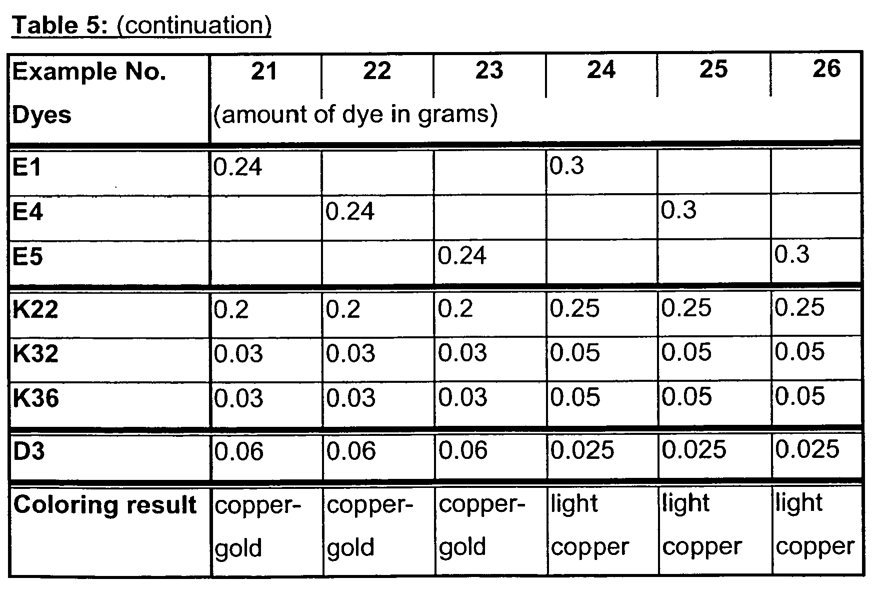

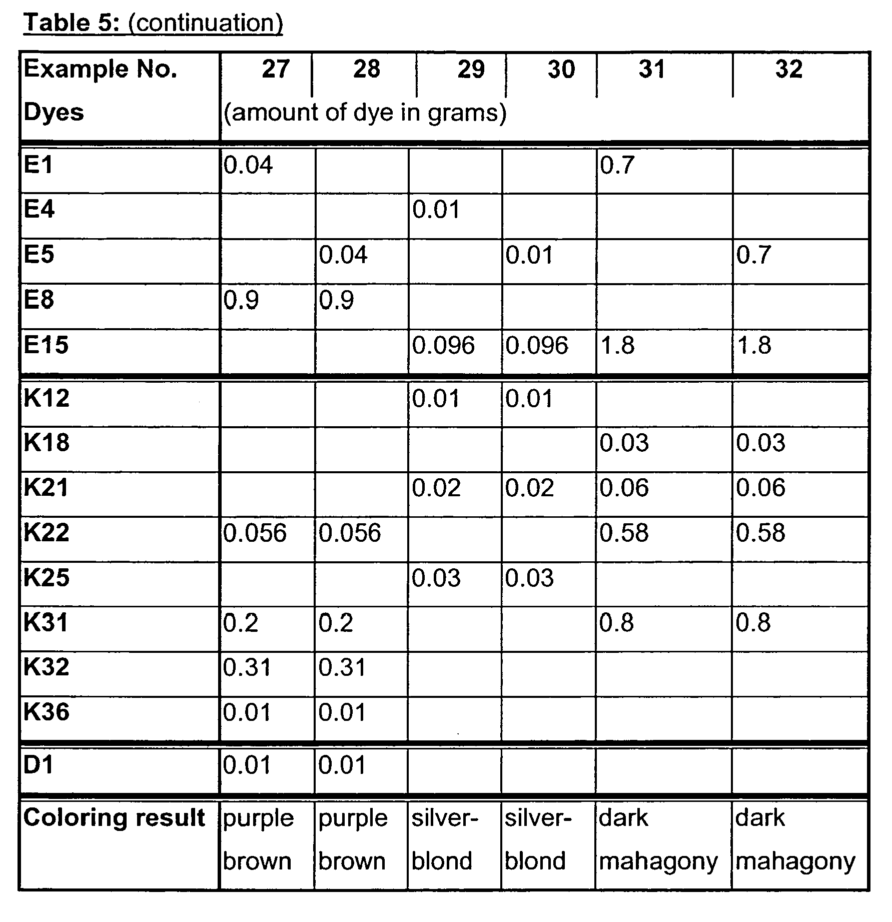

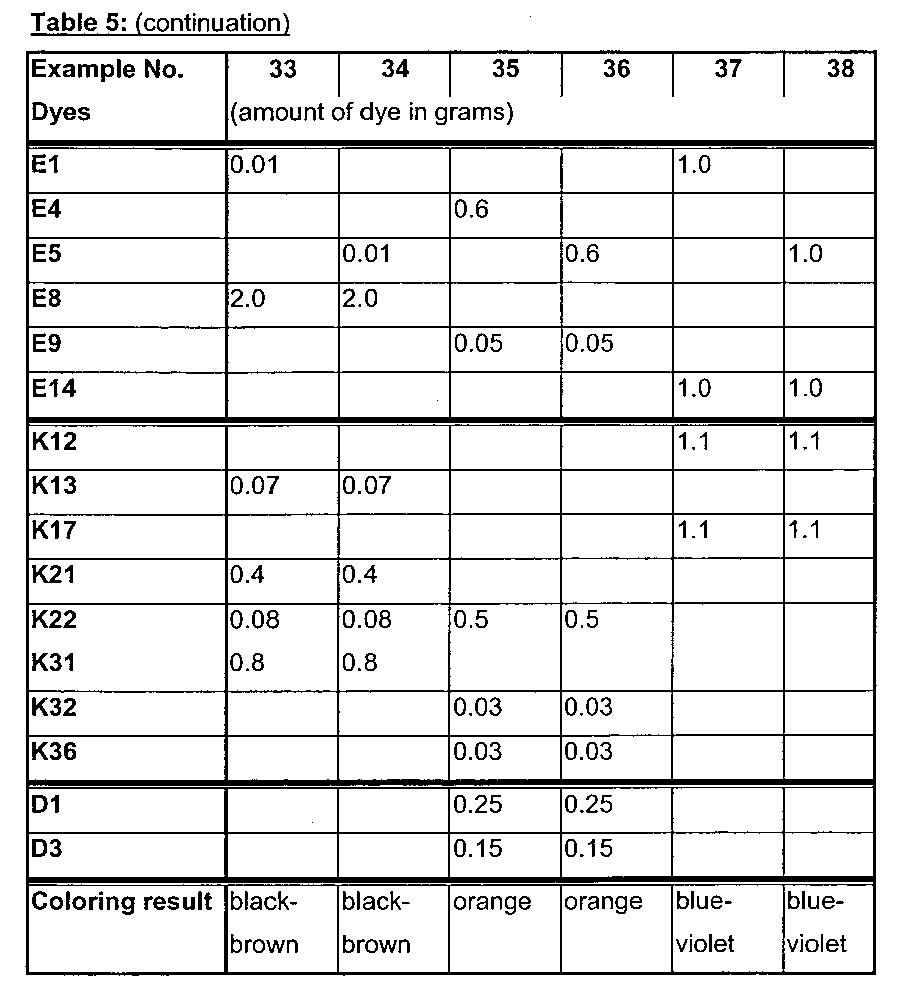

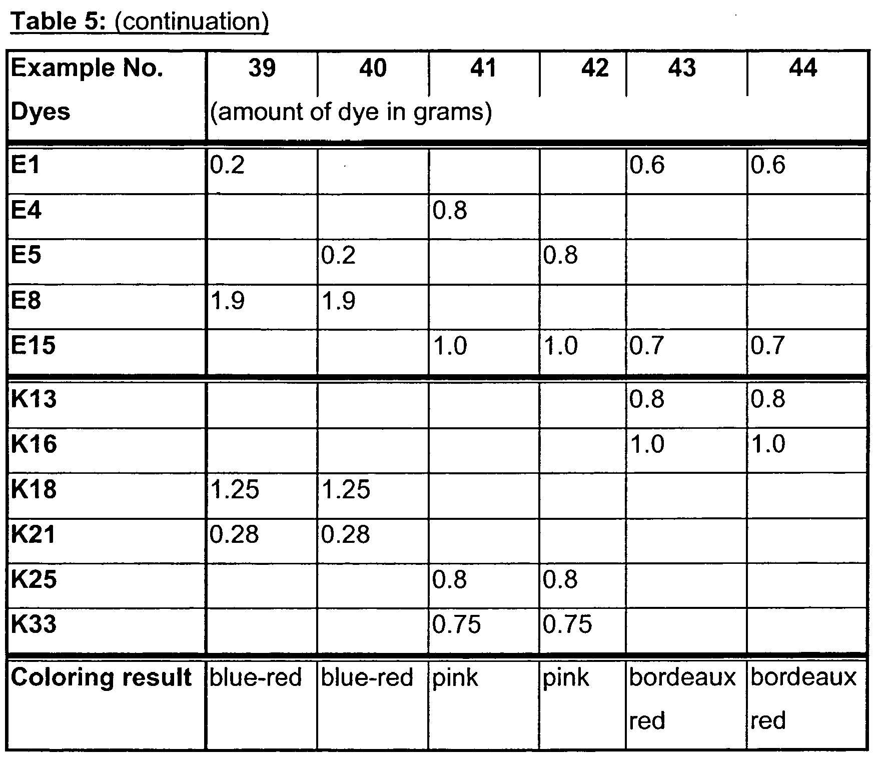

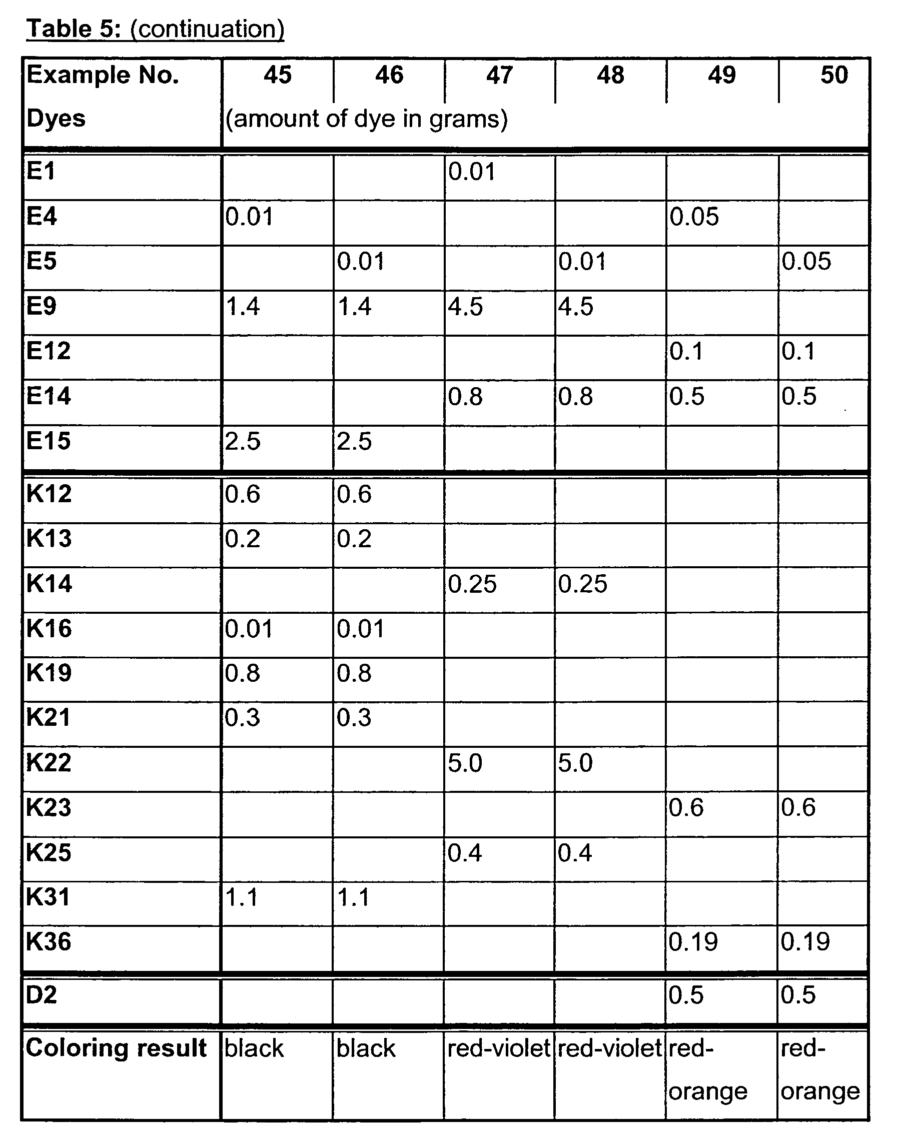

### Examples 51 to 56: Hair dyes (cream form)

- Xg: 4-aminophenol of the formula (I) as described in examples 1 to 5 (**E1** to **E5** as in table 2)
- U g: developer substance **E8** to **E15** as in table 2
- Y g: coupler substance **K12** to **K34** as in table 4
- Z g: direct dyes **D1** to **D3** as in table 3
- 3.5 g: lauryl alcohol diglycolether sulfate (28 percent aqueous solution)
- 3.0 g: ammonia (22 percent aqueous solution)
- 15.0 g: cetylalcohol
- 0.3 g: ascorbic acid
- 0.3 g: sodium sulfite
- ad 100.0 g: water, demineralized

Directly prior to application 30 g of the above coloring cream were mixed with 30 g of a 6 percent aqueous hydrogen peroxide solution. The mixture was then applied to the hair. After a contact time of 30 minutes at 40 °C, the hair was rinsed with water, washed with a standard commercial shampoo and dried. The coloring results are summarized in table 6.

**Table 6: Hair colorants**

| **Example No.** | **51** | **52** | **53** | **54** | **55** | **56** |
|---|---|---|---|---|---|---|
| **Dyes** | (amount of dye in grams) | | | | | |
| **E1** | 0.1 | | | 2.0 | | |
| **E4** | | 0.2 | | | 0.5 | |
| **E5** | | | 0.01 | | | 0.7 |
| **E10** | | | | | | 1.6 |
| **E14** | | | | 0.25 | 0.8 | 0.2 |
| **E15** | 3.2 | 1.71 | 0.02 | | | 1.8 |
| **K13** | 0.23 | 0.1 | | | 1.3 | |
| **K14** | 0.2 | | | | | |
| **K16** | | | 0.015 | | | |
| **K19** | | | | | 1.7 | |
| **K21** | 0.4 | 0.8 | | | 0.02 | |
| **K22** | 0.08 | | 0.25 | 1.8 | | 4.5 |
| **K23** | | 0.2 | | | 0.03 | |
| **K25** | | | | | | 0.55 |
| **K26** | | | 0.03 | | | |
| **K31** | 1.05 | 0.135 | 0.02 | 0.25 | | 0.8 |
| **K36** | | 0.27 | | | | |
| **D2** | | 0.01 | | | | |
| **Coloring result** | dark brown | chocolate brown | silver-blond | orange | blue-violet | red-violet |

Unless stated otherwise, all of the percentages given in the present application are percentages by weight.

## Claims

1. Agent for the oxidative dyeing of keratin fibers based on a developer substance-coupler substance combination, **characterized in that** it comprises at least one 4-aminophenol derivative of the formula (I) or its physiologically compatible, water-soluble salt, 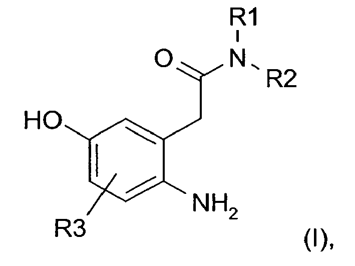 in which
**R1** and **R2,** independently of one another, are hydrogen, a saturated (C₁-C₆)-alkyl group, an unsaturated (C₂-C₆)-alkyl group, a (C₂-C₆)-hydroxyalkyl group, a (C₃-C₆)-dihydroxyalkyl group, a (C₁-C₄)-alkoxy-(C₁-C₄)-alkyl group, a (C₂-C₄)-hydroxyalkyl-(C₁-C₄)-alkoxy group, a (C₂-C₆)-aminoalkyl group, a (C₁-C₄)-alkylamino-(C₁-C₄)-alkyl group, a di(C₁-C₄)-alkylamino-(C₁-C₄)-alkyl group, a (C₂-C₆)-acetylaminoalkyl group, a (C₁-C₆)-cyanoalkyl group, a (C₁-C₆)-carboxyalkyl group, a (C₁-C₆)-aminocarbonylalkyl group, a substituted or unsubstituted phenyl group, a substituted or unsubstituted benzyl group, a pyridinylmethyl group, a furfuryl group, a hydrogenated furfuryl group, a substituted pyridinyl group, or R1 and R2, together with the nitrogen atom, form a heterocyclic ring of the formula 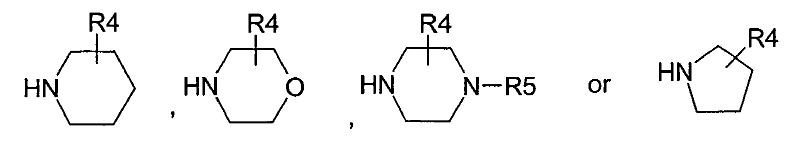 **R3** is hydrogen, a halogen atom, a C₁-C₆-alkyl group, a C₁-C₆-hydroxyalkyl group or a C₁-C₆-alkoxy group;
R4 is one or more hydrogen atoms, hydroxyl groups, carboxyl groups, aminocarbonyl groups or hydroxymethyl groups; and
**R5** is hydrogen or a (C₁-C₆)-alkyl group.

2. Agent according to claim 1, **characterized in that** in formula (I) **R3** is hydrogen.

3. Agent according to claim 1 or 2, **characterized in that** the 4-aminophenol derivative of the formula (I) is chosen from the group consisting of 2-(2-amino-5-hydroxyphenyl)acetamide, 2-(2-amino-5-hydroxyphenyl)-N-methylacetamide, 2-(2-amino-5-hydroxyphenyl)-N-ethylacetamide, 2-(2-amino-5-hydroxyphenyl)-N-propylacetamide, 2-(2-amino-5-hydroxyphenyl)-N-isopropylacetamide, 2-(2-amino-5-hydroxyphenyl)-N-butylacetamide, 2-(2-amino-5-hydroxyphenyl)-N-(2-hydroxyethyl)acetamide, 2-(2-amino-5-hydroxyphenyl)-N-(3-hydroxypropyl)acetamide, 2-(2-amino-5-hydroxyphenyl)-N-(2,3-dihydroxypropyl)acetamide, 2-(2-amino-5-hydroxyphenyl)-N-(2-methoxyethyl)acetamide, 2-(2-amino-5-hydroxyphenyl)-N-(3-methoxypropyl)acetamide, 2-(2-amino-5-hydroxyphenyl)-N-(cyanomethyl)acetamide, N-(2-aminoethyl)-2-(2-amino-5-hydroxyphenyl)-acetamide, N-(2-aminopropyl)-2-(2-amino-5-hydroxyphenyl)acetamide, 2-(2-amino-5-hydroxyphenyl)-N-cyclopentylacetamide, 2-(2-amino-5-hydroxyphenyl)-N,N-dimethylacetamide, 2-(2-amino-5-hydroxyphenyl)-N,N-diethylacetamide, 2-(2-amino-5-hydroxyphenyl)-N,N-dipropylacetamide, 2-(2-amino-5-hydroxyphenyl)-N,N-dibutylacetamide, 2-(2-amino-5-hydroxyphenyl)-N,N-bis(2-hydroxyethyl)acetamide, 2-(2-amino-5-hydroxyphenyl)-N,N-bis(2-hydroxypropyl)acetamide, 2-(2-amino-5-hydroxyphenyl)-N-(tetrahydro-2-furanylmethyl)acetamide, 2-(2-amino-5-hydroxyphenyl)-N-(2-furanylmethyl)acetamide, 2-(2-amino-5-hydroxyphenyl)-N-(2-pyridinylmethyl)acetamide, 2-(2-amino-5-hydroxyphenyl)-N-benzylacetamide, 4-amino-3-[2-oxo-2-(1-pyrrolidinyl)ethyl]phenol, 4-amino-3-[2-oxo-2-(1-piperidinyl)ethyl]phenol, 4-amino-3-[2-(4-morpholinyl)-2-oxoethyl]phenol, 4-amino-3-[2-(4-methyl-1-piperazinyl)-2-oxoethyl]phenol, 1-[(2-amino-5-hydroxyphenyl)acetyl]-3-pyrrolidinol, 1-[(2-amino-5-hydroxyphenyl)acetyl]-3-piperidinol, 2-(2-amino-5-hydroxyphenyl)-N-phenylacetamide, 2-(2-amino-5-hydroxyphenyl)-N-(4-hydroxyphenyl)acetamide, 2-(2-amino-5-hydroxyphenyl)-N-(3-hydroxyphenyl)acetamide, 2-(2-amino-5-hydroxyphenyl)-N-(2-hydroxyphenyl)acetamide, 2-(2-amino-5-hydroxyphenyl)-N-(4-methoxyphenyl)acetamide, 2-(2-amino-5-hydroxyphenyl)-N-(3-methoxyphenyl)acetamide, 2-(2-amino-5-hydroxyphenyl)-N-(2-methoxyphenyl)acetamide, 2-(2-amino-5-hydroxyphenyl)-N-[4-(hydroxymethyl)phenyl]acetamide, 2-(2-amino-5-hydroxyphenyl)-N-(2,4-dimethoxyphenyl)acetamide, 2-(2-amino-5-hydroxyphenyl)-N-[4-(dimethylamino)phenyl]acetamide, 2-(2-amino-5-hydroxyphenyl)-N-[3-(dimethylamino)phenyl]acetamide, 2-(2-amino-5-hydroxyphenyl)-N-[2-(dimethylamino)phenyl]acetamide, 2-(2-amino-5-hydroxyphenyl)-N-(4-chlorophenyl)acetamide, 2-(2-amino-5-hydroxyphenyl)-N-(3-chlorophenyl)acetamide, 2-(2-amino-5-hydroxyphenyl)-N-(2-chlorophenyl)acetamide, 2-(2-amino-5-hydroxyphenyl)-N-(4-bromophenyl)-acetamide, 2-(2-amino-5-hydroxyphenyl)-N-(3-bromophenyl)acetamide, 2-(2-amino-5-hydroxyphenyl)-N-(2-bromophenyl)acetamide, 2-(2-amino-5-hydroxyphenyl)-N-(4-fluorophenyl)acetamide, 2-(2-amino-5-hydroxyphenyl)-N-(3-fluorophenyl)acetamide, 2-(2-amino-5-hydroxyphenyl)-N-(2-fluorophenyl)acetamide, 2-(2-amino-5-hydroxyphenyl)-N-(4-methylphenyl)-acetamide, 2-(2-amino-5-hydroxyphenyl)-N-(3-methylphenyl)acetamide, 2-(2-amino-5-hydroxyphenyl)-N-(2-methylphenyl)acetamide, 2-(2-amino-5-hydroxyphenyl)-N-(4-trifluoromethylphenyl)acetamide, 2-(2-amino-5-hydroxyphenyl)-N-(3-trifluoromethylphenyl)acetamide, 2-(2-amino-5-hydroxyphenyl)-N-(2-trifluoromethylphenyl)acetamide, 2-(2-amino-5-hydroxyphenyl)-N-(4-nitrophenyl)acetamide, 2-(2-amino-5-hydroxyphenyl)-N-(3-nitrophenyl)acetamide, 2-(2-amino-5-hydroxyphenyl)-N-(2-nitrophenyl)acetamide, 2-(2-amino-5-hydroxyphenyl)-N-(4-cyanophenyl)-acetamide, 2-(2-amino-5-hydroxyphenyl)-N-(3-cyanophenyl)acetamide, 2-(2-amino-5-hydroxyphenyl)-N-(2-cyanophenyl)acetamide, 2-(2-amino-5-hydroxyphenyl)-N-(4-pyridinyl)acetamide, 2-(2-amino-5-hydroxyphenyl)-N-(3-pyridinyl)acetamide and 2-(2-amino-5-hydroxyphenyl)-N-(2-pyridinyl)-acetamide.

4. Agent according to one of claims 1 to 3, **characterized in that** it comprises the 4-aminophenol derivative of the formula (I) in an amount of from 0.005 to 20 percent by weight.

5. Agent according to one of claims 1 to 4, **characterized in that** the coupler substance is selected from the group consisting of N-(3-dimethylaminophenyl)urea, 2,6-diaminopyridine, 2-amino-4-[(2-hydroxyethyl)amino]anisole, 2,4-diamino-1-fluoro-5-methylbenzene, 2,4-diamino-1-methoxy-5-methylbenzene, 2,4-diamino-1-ethoxy-5-methylbenzene, 2,4-diamino-1-(2-hydroxyethoxy)-5-methylbenzene, 2,4-di[(2-hydroxyethyl)amino]-1,5-dimethoxybenzene, 2,3-diamino-6-methoxypyridine, 3-amino-6-methoxy-2-(methylamino)pyridine, 2,6-diamino-3,5-dimethoxypyridine, 3,5-diamino-2,6-dimethoxypyridine, 1,3-diaminobenzene, 2,4-diamino-1-(2-hydroxyethoxy)benzene, 1,3-diamino-4-(2,3-dihydroxypropoxy)benzene, 1,3-diamino-4-(3-hydroxypropoxy)benzene, 1,3-diamino-4-(2-methoxyethoxy)benzene, 2,4-diamino-1,5-di(2-hydroxyethoxy)benzene, 1-(2-aminoethoxy)-2,4-diaminobenzene, 2-amino-1-(2-hydroxyethoxy)-4-methylaminobenzene, 2,4-diaminophenoxyacetic acid, 3-[di(2-hydroxyethyl)amino]aniline, 4-amino-2-di[(2-hydroxyethyl)amino]-1-ethoxybenzene, 5-methyl-2-(1-methylethyl)phenol, 3-[(2-hydroxyethyl)amino]aniline, 3-[(2-aminoethyl)amino]aniline, 1,3-di(2,4-diaminophenoxy)propane, di(2,4-diaminophenoxy)methane, 1,3-diamino-2,4-dimethoxybenzene, 2,6-bis(2-hydroxyethyl)aminotoluene, 4-hydroxyindole, 3-dimethylaminophenol, 3-diethylaminophenol, 5-amino-2-methylphenol, 5-amino-4-fluoro-2-methylphenol, 5-amino-4-methoxy-2-methylphenol, 5-amino-4-ethoxy-2-methylphenol, 3-amino-2,4-dichlorophenol, 5-amino-2,4-dichlorophenol, 3-amino-2-methylphenol, 3-amino-2-chloro-6-methylphenol, 3-aminophenol, 2-[(3-hydroxyphenyl)-amino]acetamide, 5-[(2-hydroxyethyl)amino]-4-methoxy-2-methylphenol, 5-[(2-hydroxyethyl)amino]-2-methylphenol, 3-[(2-hydroxyethyl)amino]-phenol, 3-[(2-methoxyethyl)amino]phenol, 5-amino-2-ethylphenol, 5-amino-2-methoxyphenol, 2-(4-amino-2-hydroxyphenoxy)ethanol, 5-[(3-hydroxypropyl)amino]-2-methylphenol, 3-[(2,3-dihydroxypropyl)-amino]-2-methylphenol, 3-[(2-hydroxyethyl)amino]-2-methylphenol, 2-amino-3-hydroxypyridine, 2,6-dihydroxy-3,4-dimethylpyridine, 5-amino-4-chloro-2-methylphenol, 1-naphthol, 2-methyl-1-naphthol, 1,5-dihydroxynaphthalene, 1,7-dihydroxynaphthalene, 2,3-dihydroxynaphthalene, 2,7-dihydroxynaphthalene, 2-methyl-1-naphthol acetate, 1,3-dihydroxybenzene, 1-chloro-2,4-dihydroxybenzene, 2-chloro-1,3-dihydroxybenzene, 1,2-dichloro-3,5-dihydroxy-4-methylbenzene, 1,5-dichloro-2,4-dihydroxybenzene, 1,3-dihydroxy-2-methylbenzene, 3,4-methylenedioxyphenol, 3,4-methylenedioxyaniline, 5-[(2-hydroxyethyl)amino]-1,3-benzodioxol, 6-bromo-1-hydroxy-3,4-methylenedioxybenzene, 3,4-diaminobenzoic acid, 3,4-dihydro-6-hydroxy-1,4(2H)-benzoxazine, 6-amino-3,4-dihydro-1,4(2H)-benzoxazine, 3-methyl-1-phenyl-5-pyrazolone, 5,6-dihydroxyindole, 5,6-dihydroxyindoline, 5-hydroxyindole, 6-hydroxyindole, 7-hydroxyindole and 2,3-indolinedione.

6. Agent according to one of claims 1 to 5, **characterized in that** it additionally comprises at least one additional developer substance and/or at least one direct dye.

7. Agent according to one of claims 1 to 6, **characterized in that** the developer substances and coupler substances, based on the total amount of the colorant, are present in each case in a total amount of from 0.005 to 20 percent by weight.

8. Agent according to one of claims 1 to 7, **characterized in that** it is a hair dye.

9. 4-Aminophenol derivative of the general formula (I) or the physiologically compatible, water-soluble salt thereof, 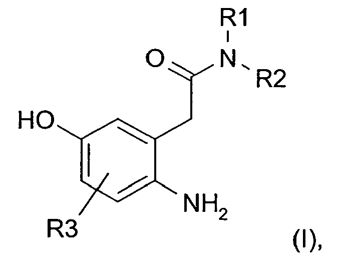 in which
**R1** and **R2,** independently of one another, are hydrogen, a saturated (C₁-C₆)-alkyl group, an unsaturated (C₂-C₆)-alkyl group, a (C₂-C₆)-hydroxyalkyl group, a (C₃-C₆)-dihydroxyalkyl group, a (C₁-C₄)-alkoxy-(C₁-C₄)-alkyl group, a (C₂-C₄)-hydroxyalkyl-(C₁-C₄)-alkoxy group, a (C₂-C₆)-aminoalkyl group, a (C₁-C₄)-alkylamino-(C₁-C₄)-alkyl group, a di(C₁-C₄)-alkylamino-(C₁-C₄)-alkyl group, a (C₂-C₆)-acetylaminoalkyl group, a (C₁-C₆)-cyanoalkyl group, a (C₁-C₆)-carboxyalkyl group, a (C₁-C₆)-aminocarbonylalkyl group, a substituted or unsubstituted phenyl group, a substituted or unsubstituted benzyl group, a pyridinylmethyl group, a furfuryl group, a hydrogenated furfuryl group, a substituted pyridinyl group, or R1 and R2, together with the nitrogen atom, form a heterocyclic ring of the formula 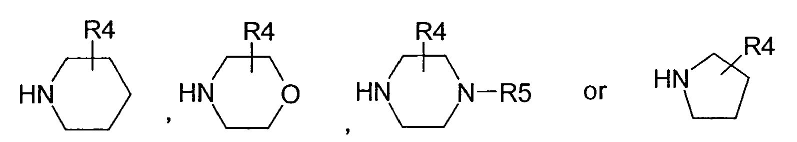 R3 is hydrogen, a halogen atom, a C₁-C₆-alkyl group, a C₁-C₆-hydroxyalkyl group or a C₁-C₆-alkoxy group;
R4 is one or more hydrogen atoms, hydroxyl groups, carboxyl groups, aminocarbonyl groups or hydroxymethyl groups; and
**R5** is hydrogen or a (C₁-C₆)-alkyl group.

10. 4-Aminophenol derivative of the general formula (I) according to claim 9, **characterized in that** in formula (I) R3 is hydrogen.
